# FASCICULE DE BREVET EUROPEEN

(11) **EP 1 335 766 B1**
(45) Date de publication et mention de la délivrance du brevet: **10.11.2004**
(21) Numéro de dépôt: 01997325.4
(22) Date de dépôt: 23.11.2001
(51) Int. Cl.: A61M 15/00

(54) **DISPOSITIF DE DISTRIBUTION DE PRODUIT FLUIDE**
FLÜSSIGKEITSABGABEVORRICHTUNG
FLUID PRODUCT DISPENSING DEVICE

(30) Priorité: 24.11.2000 FR 0015337
(43) Date de publication de la demande: 20.08.2003
(73) Titulaire: VALOIS S.A., 27110 Le Neubourg (FR)
(72) Inventeur: STRADELLA, Giuseppe, I-16032 Camogli (IT)
(74) Mandataire: CAPRI
(86) Numéro de dépôt international: PCT/FR2001/003706
(87) Numéro de publication internationale: WO 2002/041939

(56) Documents cités:
- EP-A- 0 490 797
- WO-A-98/52634
- WO-A-99/44662
- FR-A- 2 701 399
- US-A- 4 414 972
- US-A- 5 027 808
- "L'AEROSOL-DOSEUR PROLAIR AUTOHALER" ANNALES FRANCAISES DE CHRONOMETRIE ET DE MICROTECHNIQUE,FR,OBSERVATO IRE DE BESANCON. BESANCON, vol. 47, 1998, pages 115-121, XP000830922 ISSN: 0294-1228

## Description

La présente invention concerne un dispositif de distribution de produit fluide, et plus particulièrement un dispositif d'inhalation comportant une valve doseuse, du type MDI (« Metered Dose Inhaler »), dans lequel l'actionnement de la valve doseuse est commandé par l'inhalation de l'utilisateur. Voir par exemple WO-A-9944662.

De tels inhalateurs actionnes par l'inhalation, appelés généralement BAI (« Breath Actuated Inhaler »), utilisant des systèmes de valve doseuse (MDI), sont habituellement basés sur un mécanisme de déclenchement comportant un ressort, ledit ressort étant libéré par un dispositif approprié au moment de l'inhalation de l'utilisateur. Le chargement ou compression du ressort est réalisé habituellement en actionnant un levier, par exemple lors de l'ouverture du couvercle de l'embout buccal de l'inhalateur, et la force du ressort est dirigée, au moment de l'inhalation, contre la valve doseuse de l'inhalateur ou contre le réservoir, permettant l'actionnement de la valve en déplaçant la soupape de la valve par rapport au réservoir. Ceci est rendu possible du fait que l'élément parmi la soupape de la valve et le réservoir qui n'est pas soumis à l'action du ressort préalablement armé, est maintenu fixe à l'intérieur du dispositif. Ceci a pour conséquence qu'après son actionnement et suite à la distribution du produit fluide contenu dans le réservoir, la valve doseuse reste comprimée, avec la soupape dans sa position d'actionnement jusqu'à ce que la charge du ressort est relâchée, ce qui ne se produit que lorsque le couvercle de l'embout buccal est refermé.

La structure décrite ci-dessus, est la source d'un problème qui est lié à la manière dans laquelle la plupart des valves doseuse fonctionnent. Ces valves comprennent généralement un ressort de rappel et une chambre de dosage qui est remplie par le mélange constitué du produit fluide, en général un médicament, et du gaz propulseur liquéfié. Le remplissage de la chambre de dosage est réalisé par gravité et seulement lorsque la soupape de la valve se déplace de sa position de distribution vers sa position de repos, c'est à dire que la force appliquée sur la valve par le ressort du système de déclenchement est relâchée. Ceci implique donc que la tension du ressort doit être libéré lorsque le dispositif est dans une position appropriée pour permettre un remplissage par gravité de la chambre de dosage de la valve. La position requise pour un remplissage efficace et complet de la chambre de dosage est la position d'utilisation de l'inhalateur, dans laquelle le réservoir est généralement disposé au-dessus de la valve doseuse, l'utilisateur ayant l'embout buccal dans sa bouche pour inspirer la dose de produit distribué.

Dès la fin de la distribution de la dose de produit, lorsque l'utilisateur ressort le dispositif de sa bouche, la probabilité est grande que l'inhalateur ne soit plus dans la position requise pour un remplissage effectif, et le risque est grand que l'utilisateur referme le couvercle de l'embout buccal alors que le dispositif d'inhalation se trouve dans une position non appropriée pour un remplissage total de la chambre de dosage.

La présente invention a pour but de fournir un dispositif de distribution de produit fluide qui ne reproduit pas les inconvénients susmentionnés.

La présente invention a donc pour but de fournir un dispositif de distribution de produit fluide qui garantit un remplissage de la chambre de dosage de la valve dans une position appropriée pour un remplissage total et fiable de celle-ci.

La présente invention a également pour but de fournir un tel dispositif de distribution de produit fluide qui permet un remplissage de la chambre de dosage de la valve doseuse après distribution de la dose de produit, indépendamment de la position du ressort du mécanisme de déclenchement du dispositif.

La présente invention a aussi pour but de fournir un tel dispositif de distribution de produit fluide qui soit simple et peu coûteux à fabriquer et à assembler.

La présente invention a donc pour objet un dispositif de distribution de produit fluide comportant un réservoir contenant du produit fluide et un gaz propulseur, une valve doseuse, montée sur ledit réservoir, comportant une chambre de dosage et une soupape mobile entre une position de distribution et une position de repos, et un système de déclenchement automatique, de préférence actionné par l'inhalation de l'utilisateur, pour actionner ladite valve, ledit système de déclenchement comportant un élément élastique, formé par ou solidaire d'un ressort, ledit élément élastique étant déplacé manuellement par l'utilisateur vers une position armée, dans laquelle il est maintenu sous tension, l'actionnement du système de déclenchement libérant ledit élément élastique qui se déplace alors vers une position d'actionnement en exerçant une force adaptée à déplacer la soupape de la valve vers sa position de distribution, ledit élément élastique étant ensuite ramené manuellement par l'utilisateur de sa position d'actionnement vers sa position de repos, dans laquelle il ne sollicite plus ladite soupape vers sa position de distribution, caractérisé en ce que le dispositif comporte un système de libération de soupape qui coopère avec le réservoir ou la soupape de la valve de telle sorte qu'après distribution du produit contenu dans la chambre de dosage de la valve, ladite soupape est libérée de la force exercée par ledit élément élastique du système de déclenchement, de telle sorte qu'après l'actionnement du dispositif, ladite soupape est ramenée vers sa position de repos par le ressort de rappel de la valve, indépendamment de la position dudit élément élastique.

De préférence, ledit système de libération de soupape comporte un élément de blocage coopérant avec l'un parmi la soupape de la valve et le réservoir de produit, ledit élément de blocage étant mobile entre une position de blocage, dans laquelle la soupape peut être amenée dans sa position de distribution par ledit élément élastique du système de déclenchement, et une position de déblocage, dans laquelle la soupape est ramenée dans sa position de repos indépendamment de la position dudit élément élastique, ledit élément de blocage étant sollicité vers sa position de déblocage après actionnement du système de déclenchement.

Selon un premier mode de réalisation de la présente invention, le système de libération de soupape comporte un organe de retenue déplaçable entre une position de retenue dans laquelle il retient ledit élément de blocage dans sa position de blocage et une position de non-retenue, dans laquelle il ne retient pas ledit élément de blocage dans sa position de blocage, ledit organe de retenue étant déplacé vers sa position de non-retenue lorsque la soupape atteint sa position de distribution.

Avantageusement, ledit système de libération de soupape comporte un élément de commande coopérant d'une part avec la soupape de la valve et d'autre part avec ledit organe de retenue, de telle sorte que lorsque la soupape arrive dans sa position de distribution, l'élément de commande permet le déplacement de l'organe de retenue dans sa position de non-retenue, de sorte que l'élément de blocage est déplacé vers sa position de déblocage et la soupape est ramenée dans sa position de repos par le ressort de rappel de la valve.

Avantageusement, ledit organe de retenue est élastiquement déformable et ledit élément de commande comporte un premier diamètre interne coopérant avec l'organe de retenue pour empêcher sa déformation et ainsi le maintenir dans sa position de retenue, et un second diamètre interne supérieur audit premier diamètre interne, qui coopère avec ledit organe de retenue lorsque la soupape atteint sa position de distribution, permettant alors la déformation dudit organe de retenue vers sa position de non retenue.

Selon un second mode de réalisation de la présente invention, ledit élément de blocage comporte un dispositif de frein adapté à ralentir le déplacement dudit élément de blocage vers sa position de déblocage, après actionnement du système de déclenchement.

Avantageusement, ledit dispositif de frein est mécanique et comporte un élément mobile relié audit élément de blocage et coulissant à frottement contre ou entre un ou plusieurs organes de frein.

Avantageusement, ledit élément mobile est une tige, de préférence dentelée, et lesdits organes de frein comportent deux roues, de préférence en élastomère, ladite tige coulissant avec frottement entre lesdites roues.

Avantageusement, chaque roue est solidaire d'un bras déformable respectif, de telle sorte que lorsque la tige coulisse entre lesdites roues, chaque bras se déforme de telle sorte que lesdites roues se rapprochent l'une de l'autre, augmentant le frottement exercée sur ladite tige pour réaliser le freinage.

Selon une variante de réalisation dudit second mode de réalisation de l'invention, ledit dispositif de frein est pneumatique et/ou hydraulique.

Avantageusement, ledit dispositif de frein comporte un piston relié audit élément de blocage, ledit piston coulissant de manière étanche dans une chambre, ladite chambre ou ledit piston étant pourvu(e) d'un petit orifice, de sorte que du gaz ou du liquide ne peut s'écouler que lentement dans ou hors de ladite chambre, assurant un déplacement lent dudit piston.

Selon un troisième mode de réalisation de la présente invention, le système de libération de soupape comporte un premier élément de blocage déplacé vers sa position de déblocage lorsque la soupape atteint sa position de distribution, et un second élément de blocage pourvu d'un système de frein, et sollicité vers sa position de déblocage par ledit premier élément de blocage lorsqu'il est en position de déblocage, de sorte que le système de frein n'est actionné qu'à partir du moment où la soupape est dans sa position de distribution.

Avantageusement, ledit premier élément de blocage coopère d'une part avec le fond du réservoir et d'autre part avec ledit second élément de blocage, solidaire dudit frein.

Selon un quatrième mode de réalisation de la présente invention, ledit système de libération de soupape comporte un système de retardateur adapté à libérer la soupape de la valve doseuse après un temps de retardement prédéterminé après que la soupape a atteint sa positon de distribution.

Avantageusement, le système de libération de soupape comporte un premier élément de blocage retenu par un organe de retenue lui-même coopérant avec un élément de commande, ledit élément de commande comportant une première partie solidaire de l'élément élastique du système de déclenchement, et une seconde partie coopérant d'une part avec ledit organe de retenue, et d'autre part avec un second élément de blocage pourvu d'un système de frein, un organe élastique de faible raideur, tel qu'un ressort, étant interposé entre lesdites première et seconde parties de l'élément de commande, de sorte que ledit premier élément de blocage ne peut se déplacer vers sa position de déblocage qu'après un temps de retardement prédéterminé pour permettre à ladite soupape de la valve de retourner vers sa position de repos, ledit temps de retardement correspondant au temps nécessaire audit second élément de blocage pour se déplacer vers sa position de déblocage, contre ledit système de frein, sous l'effet dudit organe élastique, pour déplacer ainsi ladite seconde partie de l'élément de commande dans la position dans laquelle l'organe de retenue peut se déformer dans sa position de non-retenue, pour permettre au premier élément de blocage de se déplacer vers sa position de déblocage.

En variante, ledit système retardateur est électronique ou électro-mécanique.

D'autres caractéristiques et avantages de la présente invention apparaîtront plus clairement au cours de la description détaillée suivante de plusieurs modes de réalisation de celle-ci, décrits en référence aux dessins joints, et donnés à titres d'exemples non limitatifs, sur lesquels :
- la figure 1 est une vue schématique en section transversale partiellement découpée d'un dispositif de distribution de produit fluide selon un premier mode de réalisation de la présente invention, avant actionnement de la valve,
- la figure 2 est une vue similaire à celle de la figure 1, après actionnement de la valve,
- la figure 3 est une vue schématique en section transversale partiellement découpée d'une variante de réalisation du premier mode de réalisation de l'invention, avant actionnement de la valve,
- la figure 4 est une vue similaire à celle de la figure 3, après actionnement de la valve,
- la figure 5 est une vue schématique en section transversale d'un dispositif de distribution de produit fluide selon un second mode de réalisation de la présente invention, avant actionnement de la valve,
- la figure 6 est une vue de détail agrandie du système de frein représenté sur la figure 5,
- la figure 7 est une vue schématique en section transversale dudit système de frein,
- la figure 8 représente une variante de réalisation dudit second mode de réalisation de l'invention, avant actionnement de la valve,
- la figure 9 est une vue de détail schématique en section du système de frein pneumatique de la figure 8, avant actionnement du dispositif,
- la figure 10 est une vue similaire à celle de la figure 9 pendant l'actionnement du dispositif,
- la figure 11 est une vue similaire aux figures 9 et 10, après actionnement du dispositif,
- la figure 12 est une vue schématique en section d'un troisième mode de réalisation de la présente invention, qui combine les premier et second modes de réalisation,
- la figure 13 est une vue schématique en section d'une variante de réalisation du système de frein pneumatique, avant actionnement,
- la figure 14 est une vue similaire à la figure 13, après actionnement,
- la figure 15 est une vue schématique en section d'un quatrième mode de réalisation de la présente invention, avant actionnement,
- la figure 16 est une vue similaire à la figure 15 pendant l'actionnement, et
- la figure 17 est une vue similaire aux figures 15 et 16, après actionnement du dispositif.

La présente invention s'applique à tous types d'inhalateurs déclenchés par l'inhalation de l'utilisateur (BAI), et même si la description de plusieurs modes de réalisation va être faite en connexion avec un tel inhalateur dans lequel le ressort du système de déclenchement par l'inhalation agit sur la soupape de la valve, il est clair qu'elle s'applique également aux dispositifs dans lesquels le ressort agit sur le fond du réservoir. Que la force élastique d'actionnement de la valve soit exercée sur la soupape ou sur le réservoir n'a pas d'influence directe sur la présente invention qui s'applique dans les deux cas, l'objet de la présente invention étant de permettre un retour de la soupape vers sa position de repos indépendamment de la position ou de l'état dudit ressort du système de déclenchement par l'inhalation.

La description ci-après va donc être faite en référence à un dispositif du type divulgué dans le document WO 99/44662, ce document étant incorporé à titre de référence dans la présente invention en ce qui concerne le fonctionnement du système de déclenchement par l'inhalation du dispositif de distribution de produit fluide.

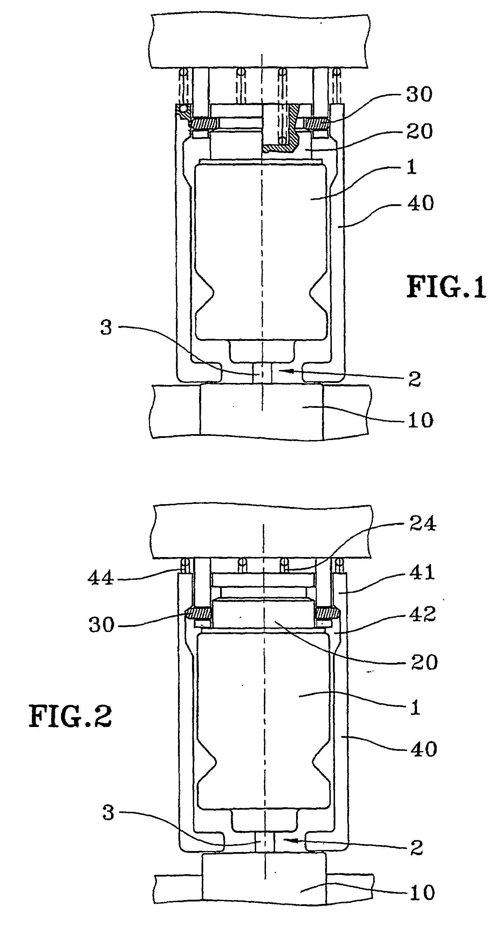

En référence aux figures 1 et 2, il va être décrit un premier mode de réalisation de l'invention. Dans ce premier mode de réalisation, on prévoit un système de libération de soupape qui agit automatiquement au moment où la soupape atteint sa position de distribution, dans laquelle elle expulse la dose de produit contenu dans la chambre de dosage.

La figure 1 représente très schématiquement un réservoir de produit fluide 1 sur lequel est monté une valve doseuse 2 d'une manière quelconque souhaitée, ladite valve doseuse 2 comprenant une soupape 3 mobile entre une position de repos et une position de distribution. La valve doseuse 2 comporte une chambre de dosage (non représentée) qui est vidée lorsque la soupape 3 est dans sa position de distribution et qui se remplit par gravité lorsque la soupape 3 revient de sa position de distribution vers sa position de repos. Ladite soupape 3 coopère avec un élément élastique 10 qui fait partie d'un système de déclenchement par l'inhalation et qui est constitué par ou est solidaire d'un ressort (non représenté sur les figures 1 et 2), ledit ressort étant armé par l'utilisateur avant l'utilisation du dispositif de sorte que lors de l'inhalation, l'élément élastique 10 est libéré et peut exercer une force sur la soupape 3 pour actionner la valve doseuse. Pendant ce processus pendant lequel la soupape 3 se déplace de sa position de repos vers sa position d'actionnement, le réservoir 1 est maintenu fixe dans le corps du dispositif.

Le système de libération de soupape comporte un élément de blocage 20, qui dans l'exemple des figures 1 et 2 coopère avec le fond du réservoir 1. Cet élément de blocage 20 est retenu dans sa position de blocage par un organe de retenue 30, qui dans l'exemple représenté sur les figures 1 et 2 est réalisé sous la forme d'un anneau fendu pouvant se déformer radialement vers l'extérieur. En variante, l'organe de retenue pourrait être réalisé sous la forme d'une ou plusieurs pattes élastiques déformables vers l'extérieur. Cet anneau fendu 30 coopère avec ledit élément de blocage 20 pour le maintenir dans sa position de blocage, dans laquelle il maintient le réservoir 1 fixe à l'intérieur du dispositif. Un élément de commande 40 est relié d'une part à l'élément élastique 10 du système de déclenchement par l'inhalation et coopère d'autre part avec ledit élément de retenue 30. Ainsi, comme visible sur les figures 1 et 2, l'élément de commande 40 est déplacé en même temps que l'élément élastique 10, et donc en même temps que la soupape 3 lorsque la valve doseuse est actionnée. Il est réalisé sous la forme d'un manchon qui entoure de manière externe ledit anneau 30 et comporte un premier diamètre 41 et un second diamètre 42 supérieur audit premier diamètre. Le premier diamètre 41 de l'élément de commande 40 coopère avec l'organe de retenue 30 avant l'actionnement du dispositif, et le second diamètre 42 coopère avec ledit organe de retenue 30 après l'actionnement du dispositif, lorsque la soupape 3 est dans sa position de distribution. A ce moment là, l'organe de retenue 30 peut se déformer radialement vers l'extérieur à l'intérieur du second diamètre 42 de l'élément de commande 40 pour libérer l'élément de blocage 20. En variante, la partie de second diamètre de l'élément de commande peut être réalisée sous la forme d'une ouverture, l'essentiel étant que l'organe de retenue puisse se déformer vers sa position de non retenue. L'élément de blocage 20 qui peut alors coulisser axialement sous l'effet de la force exercée par le ressort de rappel (non représenté) de la valve doseuse, de sorte que celle-ci revient vers sa position de repos dès que l'organe de retenue 30 est déplacé vers sa position de non retenue représentée sur la figure 2. La soupape 3 est toujours bloquée par l'élément élastique 10 du système de déclenchement, tant que celui-ci n'est pas ramené manuellement par l'utilisateur vers sa position de repos, mais c'est le réservoir 1 qui peut alors librement se déplacer pour permettre à la soupape 3 de revenir vers sa position de repos après distribution du produit. C'est donc l'élément qui est fixe lors de l'actionnement, en l'occurrence le réservoir dans cet exemple, qui se déplace pour libérer la soupape.

Ainsi, dans l'exemple de réalisation représenté sur les figures 1 et 2, au moment où la soupape 3 atteint sa position de distribution et délivre le produit contenu dans la chambre de dosage de la valve doseuse 2, le réservoir 1 est libéré, l'élément de blocage 20 pouvant se déplacer vers sa position de déblocage, la soupape 3 revenant alors vers sa position de repos, ce qui permet un remplissage de la chambre de dosage alors que le dispositif est toujours dans la bouche de l'utilisateur, et garantissant que ce remplissage se produit dans la position requise, comme représentée sur les dessins, avec la valve doseuse 2 disposée en dessous du réservoir 1, le remplissage étant réalisé par gravité.

De manière avantageuse, le remplissage est réalisé dès la fin de la distribution de la dose précédente, c'est à dire très rapidement. Ceci permet d'éviter tout problème de surdosage qui pourrait se produire en cas d'attente en position inversée, notamment avec des suspensions.

Avantageusement, on peut prévoir un ressort de rappel 24 pour l'élément de blocage 20 et un ressort de rappel 44 pour l'élément de commande 40, de sorte que lorsque l'utilisateur ramène manuellement l'élément élastique 10 vers sa position de repos, l'élément de commande 40 est ramené automatiquement vers sa position initiale par ledit ressort de rappel 44, de même que l'élément de blocage 20 est ramené vers sa position de blocage par le ressort de rappel 24, l'organe de retenue 30 revenant se positionner à l'intérieur de la rainure dudit élément de blocage 20 pour bloquer ledit élément de blocage en position de blocage, et le premier diamètre 41 de l'organe de commande vient bloquer l'organe de retenue en position de retenue.

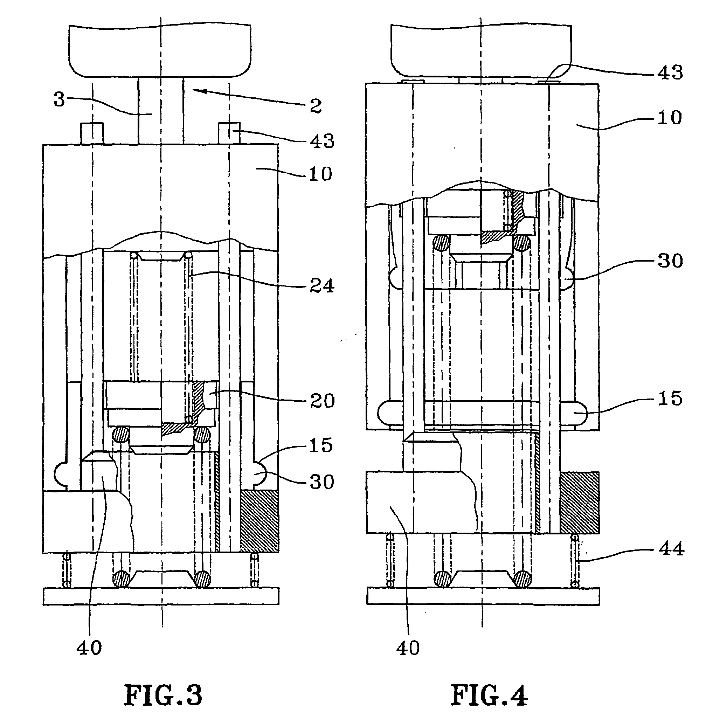

En référence aux figures 3 et 4, il est représenté une variante de réalisation du mode de réalisation représenté sur les figures 1 et 2. Dans cette variante, le système de libération de soupape n'agit pas sur le fond du réservoir 1 mais coopère directement d'une part avec l'élément élastique 10 du système de déclenchement par inhalation et d'autre part avec la soupape 3. Dans ce cas, l'élément élastique 10 est au moins partiellement creux et incorpore un élément de blocage 20 qui est pourvu de bras flexibles comportant à l'extrémité des projections radiales 30 formant l'organe de retenue du système de libération de soupape. Les projections 30, et donc l'élément de blocage 20, sont maintenus en position de blocage par l'élément de commande 40 qui verrouille les bras flexibles dans une position droite dans laquelle les projections 30 sont forcées en prise avec une rainure 15 formée dans les parois internes de l'élément élastique 10. Cet élément de commande 40 comporte une ou plusieurs tiges 43 qui passent à travers des ouvertures appropriées réalisées dans l'élément de blocage 20 et l'élément élastique 10 du système de déclenchement par inhalation et qui font saillie à partir dudit élément élastique 10 en direction du réservoir 1 et de la valve doseuse 2.

Ainsi, dans cette variante, le système de libération de soupape est réalisé à l'intérieur dudit élément élastique 10 du système de déclenchement par inhalation. A la fin de la course de la soupape 3, lorsque celle-ci arrive en position de distribution et distribue la dose de produit contenu dans la chambre de dosage, les extrémités des tiges 43 atteignent le col du réservoir ou la bague de fixation de la valve doseuse sur ledit réservoir, et sont déplacées par celui-ci dans la direction vers le bas sur les figures 3 et 4. Ceci entraîne l'élément de commande 40 à se déplacer de sa position représentée sur la figure 3 vers sa position représentée sur la figure 4 dans laquelle il ne coopère plus avec les bras élastiques de l'élément de blocage 20 et donc avec les projections 30 à l'extrémité desdits bras, de sorte que ledit élément de blocage 20 est libéré et peut se déplacer vers sa position de déblocage par flexion desdits bras pour supprimer l'interaction entre les projections 30 et la rainure 15 correspondante ménagée dans la paroi interne de l'élément élastique 10. Le résultat est que l'élément de blocage 20 est projeté vers le haut sur les figures 3 et 4, libérant ainsi la tension appliquée sur le ressort de l'élément élastique 10. Ainsi, la soupape 3 de la valve doseuse peut revenir vers sa position de repos sous l'effet du ressort de rappel de la valve.

Lorsque l'utilisateur ramène ensuite l'élément élastique 10 vers sa position de repos, notamment en refermant le couvercle de l'embout buccal (non représenté), l'élément de blocage 20 reprend sa position d'origine représentée sur la figure 3, grâce au ressort de rappel 24 prévu à cet effet. Pendant que cet élément de blocage 20 revient vers sa position de repos il pousse vers le bas sur les figures 3 et 4 l'élément de commande 40 qui alors atteint également à nouveau la position de repos représentée sur la figure 3 dans laquelle les projections 30 reviennent coopérer avec la rainure 15 correspondante, grâce au ressort de rappel 44 prévu à cet effet.

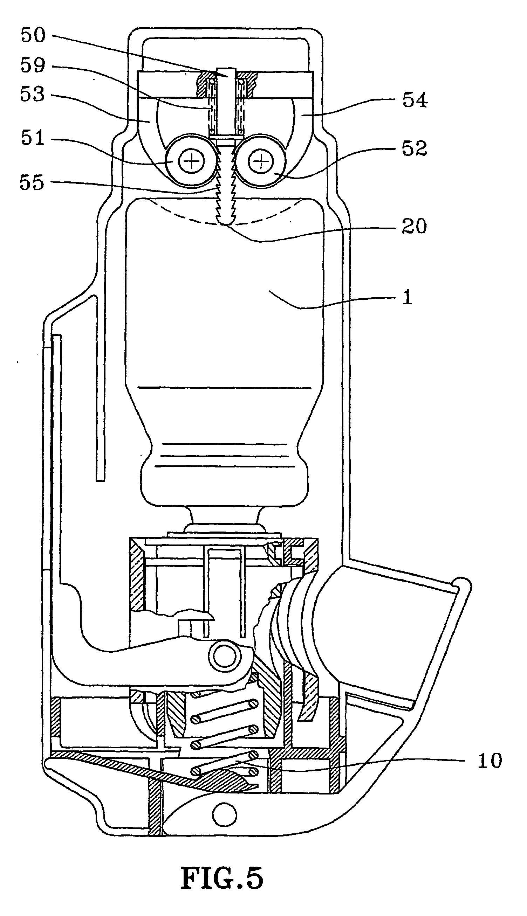

En référence aux figures 5, 6 et 7, nous allons décrire un second mode de réalisation de l'invention. Dans ce second mode de réalisation, on prévoit un système de libération de soupape qui comporte un dispositif de frein agissant sur l'élément parmi la soupape de la valve et le réservoir qui n'est pas soumis à la force élastique de l'élément élastique 10 du système de déclenchement par l'inhalation, c'est à dire sur l'élément qui reste fixe lors de l'actionnement. Ce dispositif de frein permet un déplacement lent et prédéterminé de la partie du BAI normalement maintenu fixe pendant l'actionnement de la valve. L'accélération fournie par ce dispositif de frein est nettement inférieure à l'accélération du déplacement de la soupape par rapport au réservoir lors de l'actionnement de la valve doseuse sous l'effet de l'élément élastique du système de déclenchement par l'inhalation, de sorte que la soupape est très rapidement amenée dans sa position de distribution pour distribuer le produit fluide contenu dans la chambre de dosage de la valve, alors que le déplacement lent prédéterminé susmentionné permet une libération de la soupape dans un temps prédéterminé qui est dépendant de l'efficacité du dispositif de frein, et indépendant de la position de l'élément élastique du système de déclenchement par l'inhalation.

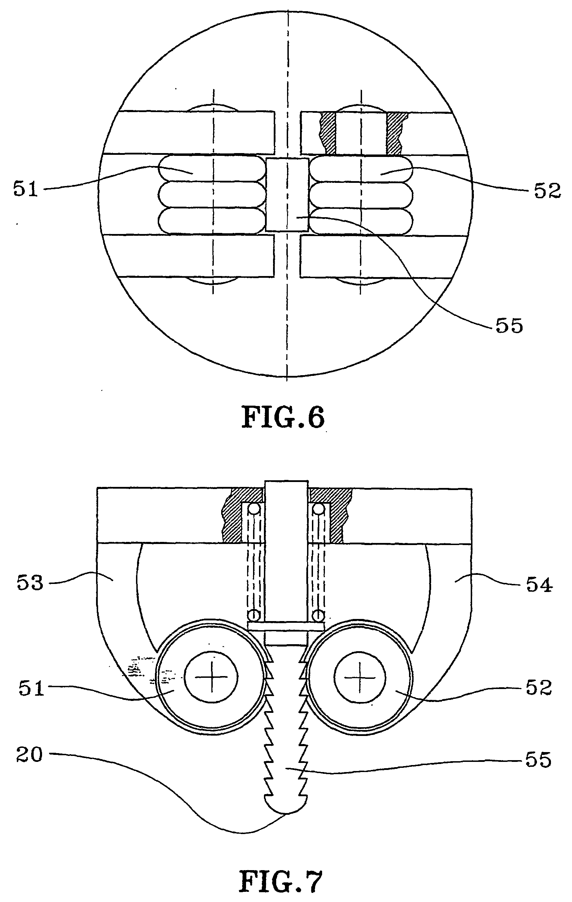

En référence à la figure 5, il est représenté un exemple de réalisation d'un dispositif de frein 50 du type mécanique. Ce frein 50, qui coopère ici avec le fond du réservoir 1, comporte un élément mobile tel qu'une tige 55 dont l'extrémité en contact avec le fond du réservoir 1 forme l'élément de blocage 20. Cette tige 55 est de préférence dentelée, et peut coulisser entre deux roues de frottement 51 et 52, qui sont de préférence réalisées en matériau élastomère. Avantageusement, comme visible plus particulièrement sur la figure 7, chaque roue de frottement 51, 52 est solidaire d'un bras semi-rigide respectif 53 et 54 qui peut se fléchir vers l'intérieur pour ainsi provoquer un rapprochement des deux roues 51 et 52 l'une vers l'autre. Ainsi, lorsque l'utilisateur actionne le dispositif de distribution de produit fluide, en déclenchant le système de déclenchement par l'inhalation, l'ensemble formé du réservoir et de la valve doseuse 2 est poussée contre le frein 50 par l'élément élastique 10 du système de déclenchement, ledit fond du réservoir étant retenu par l'élément de blocage 20 formé par l'extrémité de la tige 55. Celle-ci est donc poussée entre les deux roues 51 et 52. La présence des dents sur la tige 55 favorise la flexion des bras flexibles 53 et 54 et donc le rapprochement des deux roues 51 et 52 l'une vers l'autre, pour augmenter la force de frottement exercée sur la tige 55, ce qui a pour effet de réaliser le freinage souhaité. Le déplacement du réservoir 1 lors de la poussée exercée par l'élément élastique 10 du système de déclenchement est donc freiné par le frein 50, de sorte que la soupape 3 de la valve 2 atteint d'abord très rapidement sa position de distribution pour distribuer le produit, alors que le réservoir 1 se déplace beaucoup plus lentement en fin de déplacement de la tige 55 entre les deux roues 51 et 52, donc en position de déblocage de l'élément de blocage 20, la soupape 3 est ramenée vers sa position de repos et de remplissage sous l'effet du ressort de rappel (non représenté) de la valve doseuse. Grâce au frein 50, ce retour automatique de la soupape 3 vers sa position de repos n'est pas réalisé immédiatement après la distribution du produit fluide, mais avec un certain délai correspondant à l'action du frein 50 sur le fond du réservoir 1.

Avantageusement, on prévoit également un ressort de rappel 59 pour ramener le frein 50 dans sa position de départ lorsque l'élément élastique 10 du système de déclenchement par l'inhalation est ramené dans sa position de repos, notamment par fermeture du capot de l'embout buccal. La forme des dents de la tige dentelée 55 est de préférence réalisée de telle sorte que lorsqu'elle se déplace vers sa position de déblocage, elle entraîne les roues en élastomère 51 et 52 à se rapprocher l'une de l'autre sous l'effet de la flexion des bras flexibles 53 et 54 respectivement, pour augmenter la force de frottement. Au contraire, quand la tige revient vers sa position de blocage sous l'effet du ressort de rappel 59, ladite tige dentelée glisse aisément entre lesdites deux roues 51 et 52, les dents ayant même tendance à les écarter pour faciliter ce retour en position de départ.

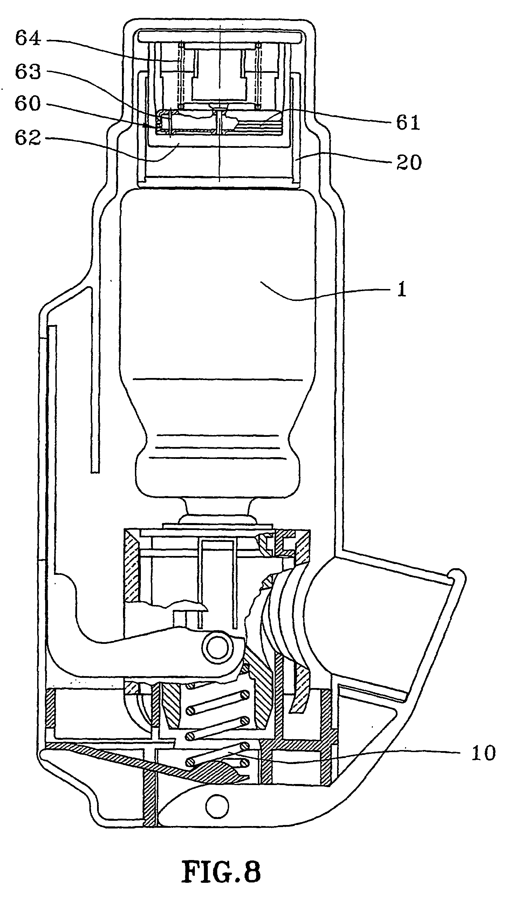

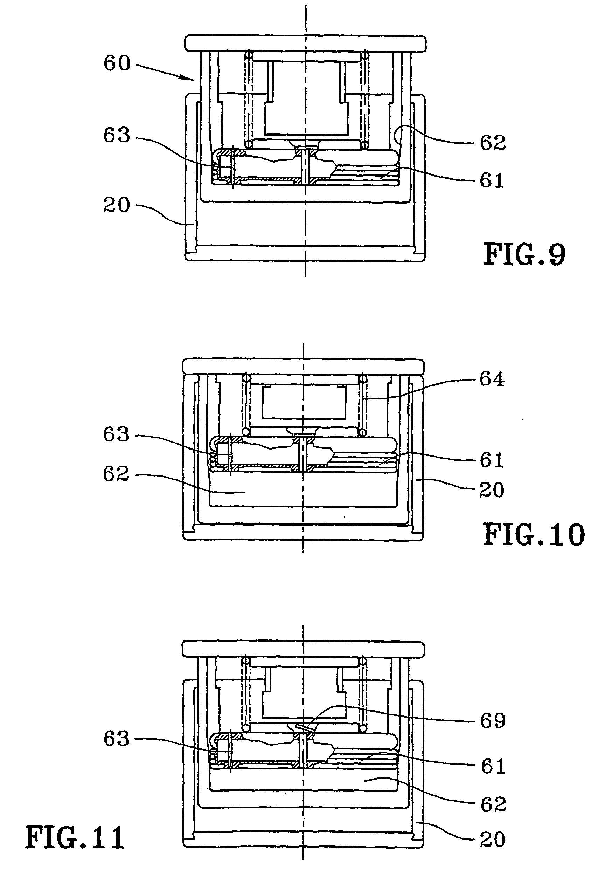

En référence aux figures 8 à 11, nous allons maintenant décrire une autre variante de réalisation dudit frein décrit ci-dessus, dans laquelle le frein n'est plus un frein mécanique mais un frein pneumatique 60. Ce frein 60 comporte un piston 61 relié à un élément de blocage 20 qui coopère avec le fond du réservoir 1. Ledit piston 61, qui comporte de préférence un revêtement en élastomère, coulisse de manière étanche dans une chambre 62, ladite chambre 62 ou ledit piston 61 étant pourvus d'un petit orifice 63 de faible diamètre. Dans l'exemple représenté sur les figures 8 à 11, le frein 60 fonctionne par dépression, c'est à dire que dans la position de repos représentée sur les figures 8 et 9, le piston 61 est disposé contre la paroi d'extrémité formant la chambre 62. Lorsque l'utilisateur actionne le dispositif et que le réservoir 1 transmet la pression exercée par l'élément élastique 10 du système de déclenchement sur l'élément de blocage 20, celui-ci entraîne le piston à se déplacer en éloignement de la paroi d'extrémité de la chambre 62, créant une dépression entre cette paroi d'extrémité et ledit piston 61, l'orifice 63 de petit diamètre ne permettant une pénétration d'air à l'intérieur de ladite chambre 62 qu'à faible vitesse, de sorte que ledit piston 61 et donc l'élément de blocage 20 ne peuvent se déplacer que lentement, assurant ainsi le freinage requis. Bien entendu, l'efficacité du frein dépend des dimensions de l'orifice 63 et de la chambre 62.

Avantageusement, le frein pneumatique est également pourvu d'un ressort de rappel 64 et d'un clapet anti-retour 69 qui permet un retour rapide du piston dans sa position de départ lorsque l'élément élastique du système de déclenchement est ramené manuellement par l'utilisateur dans sa position de repos (notamment par fermeture du couvercle).

Bien que l'exemple du frein pneumatique a été décrit en référence à un système fonctionnant à dépression, il est évident que le frein pneumatique des figures 8 à 11 peut être réalisé de manière à fonctionner par compression. Dans ce cas, le piston 61 est dans la position de repos en éloignement de la paroi d'extrémité de la chambre 62 et lorsque le dispositif est actionné, il est sollicité élastiquement en direction de cette paroi d'extrémité de sorte que l'air contenu à l'intérieur de la chambre 62 ne peut s'échapper qu'à travers ledit orifice de petit diamètre 63, ce qui ne peut se faire que lentement ce qui ainsi fournit le freinage requis.

D'autre part, on peut également envisager de réaliser le système de frein de manière hydraulique, en remplaçant l'air par un liquide quelconque souhaité, et en adaptant de manière correspondante les dimensions de la chambre 62 et de l'orifice de petit diamètre 63.

En variante, on pourrait aussi utiliser un système d'engrenage permettant de fournir la fonction de freinage souhaitée.

Le concept du frein, dont un exemple a été décrit ci-dessus, est approprié à la condition que la résistance du ressort de rappel (non représenté) de la valve doseuse n'est pas trop élevée et par conséquent la charge de l'élément élastique 10 du système de déclenchement par inhalation, qui au final agit contre le frein 50 n'est pas trop élevée. En effet, plus la force appliquée sur le frein est élevée moins son efficacité est grande.

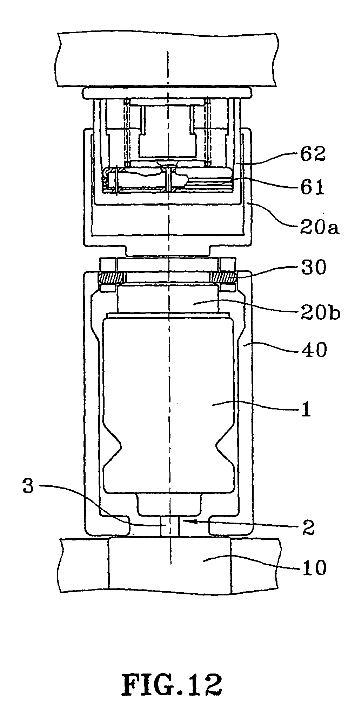

En référence maintenant à la figure 12, il va être décrit un troisième mode de réalisation de la présente invention, dans lequel on combine à la fois le système décrit dans le second mode de réalisation ci-dessus, à savoir le frein, et le système décrit dans le premier mode de réalisation, à savoir un système de libération de soupape agissant à partir du moment où la soupape 3 de la valve arrive dans sa position de distribution. Cette combinaison permet d'éviter que le frein fonctionne dès le début de la course d'actionnement de la soupape de la valve doseuse, ledit frein étant actionné par ledit système de libération de soupape seulement au moment où la soupape atteint sa position de distribution. Ainsi, pendant la course d'actionnement de la soupape entre sa position de repos et sa position d'actionnement, le frein n'est pas encore actif, et le réservoir reste fixe.

En référence à la figure 12, il va être décrit une première variante de réalisation d'une telle combinaison. Ainsi, c'est un premier élément de blocage 20a qui coopère avec le fond du réservoir 1 et qui n'est libéré de sa position de blocage et déplacé vers sa position de déblocage que lorsque l'élément de retenue 30 est libéré de sa position de retenue. Ceci se produit lorsque l'élément de commande 40 arrive en position de libération, qui correspond à la position de distribution de la soupape 3. Au moment où la soupape 3 arrive donc dans sa position de distribution et distribue le produit contenu dans la chambre de dosage, le premier élément de blocage 20a est libéré et peut se déplacer axialement vers le haut sur la figure 12 sous l'effet du ressort de rappel de la valve doseuse 2, mais ce déplacement est freiné puisque le premier élément de blocage 20a coopère avec le second élément de blocage 20b, qui lui forme partie du frein pneumatique 60 décrit précédemment en étant relié au piston 61 qui coulisse dans la chambre 62. Le retour de la soupape 3 vers sa position de repos est donc réalisé lentement, ce qui permet de garantir la distribution totale du produit contenu dans la chambre de dosage. Ceci permet de ne pas forcément utiliser une valve doseuse extrêmement rapide, comme cela est le cas lorsque le système de libération de soupape en fin de course d'actionnement de la soupape est utilisé seul.

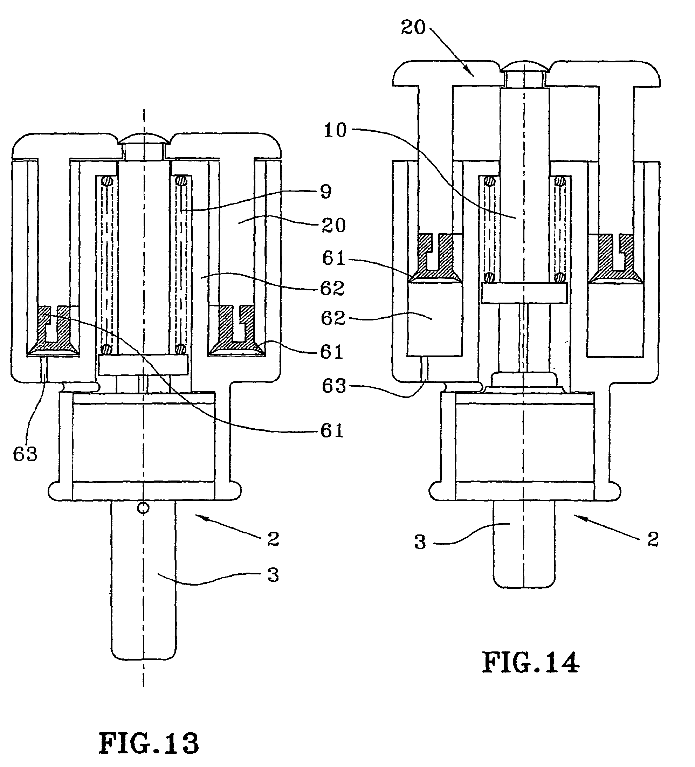

En référence aux figures 13 et 14, il est représenté un système de frein hydraulique qui est appliqué directement sur la valve doseuse 2 et qui peut être utilisé dans le cadre d'une combinaison telle que décrite en référence sur la figure 12, avec un système de libération de soupape agissant lorsque la soupape atteint sa position de distribution. Dans cet exemple de réalisation représenté sur les figures 13 et 14, la valve 2 est du type standard et le corps de valve a été modifié pour former une chambre annulaire 62 qui loge un piston annulaire 61 pourvu d'une lèvre d'étanchéité et qui peut coulisser de manière étanche dans ladite chambre 62. La chambre 62 est pourvue d'un petit trou de faible diamètre 63 et le piston 61 est connecté à la tige de soupape, que nous avons ici référencée par la référence numérique 10, puisque l'élément élastique 10 du système de déclenchement par l'inhalation, qui agit sur la soupape au moment de la distribution, en transmettant sa force directement à la soupape 3 et donc à la tige de soupape 10 relié au piston 61 n'est pas représenté sur les figures 13 à 14. La tige de la soupape 10 est également connectée à l'élément de blocage 20 qui forme en fait la liaison entre la tige de soupape 10 et le piston 61. La tige de soupape 10 est également reliée directement au ressort de rappel 9 de la valve doseuse 2, de manière classique.

Ainsi, pendant l'actionnement de la valve doseuse 2, le piston 61 coulisse vers le haut sur les figures 13 et 14 dans la chambre 62 sans aucune difficulté, la lèvre d'étanchéité du piston agissant en tant que clapet anti-retour qui permet au produit fluide contenu dans la valve doseuse de pénétrer sans aucune difficulté à l'intérieur de la chambre 62. Lorsque la soupape 3 est ramenée vers sa position de repos, ce qui est rendu possible à partir du moment où elle a atteint sa position de distribution, au moyen du premier système de libération de soupape qui agit à partir de cette position comme décrit précédemment, le piston 61 est donc forcé en retour vers sa position de repos représenté sur la figure 13 par le ressort de rappel 9 de la valve doseuse, mais il doit surmonter la résistance du liquide qui soit s'échapper de la chambre 62 à travers l'orifice de petit diamètre 63, puisque dans sa course de retour, le piston 61 coulisse de manière étanche dans ladite chambre 62. L'effet de freinage requis est donc réalisé ici de manière très simple directement dans la valve doseuse. Il est à noter que cette valve pourrait être mise en oeuvre indépendamment du système de déclenchement par l'inhalation.

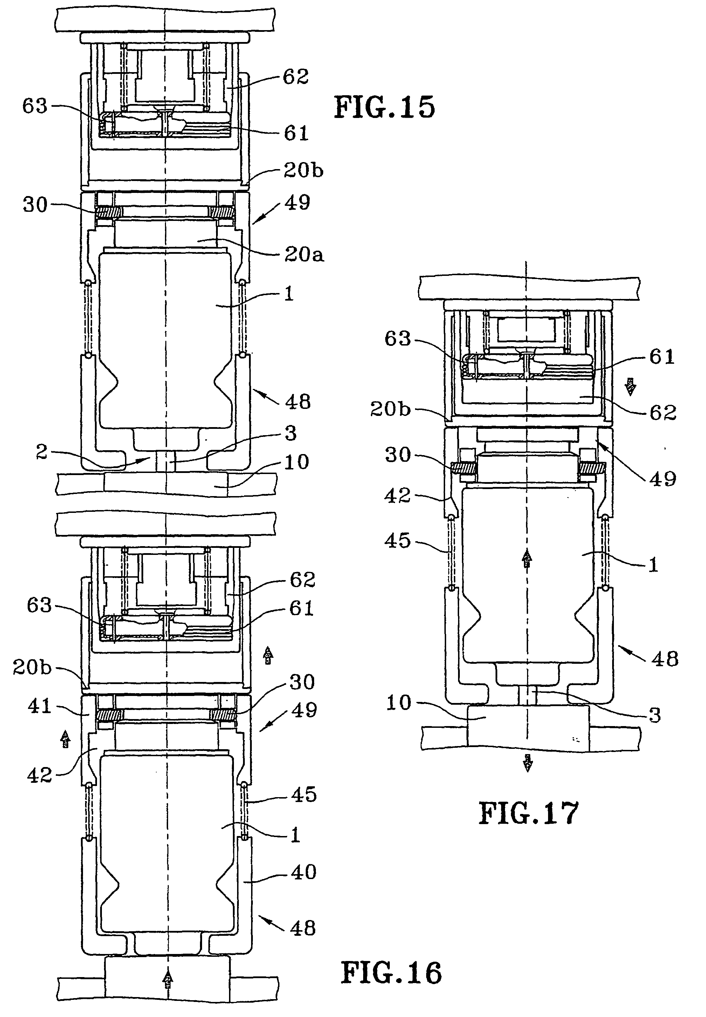

En référence aux figures 15 à 17, il est représenté un quatrième mode de réalisation de la présente invention, dans lequel on prévoit d'utiliser un "système retardateur", ledit retardateur retardant en fait d'un temps prédéterminé l'actionnement du système de libération de soupape décrit en référence aux figures 1 et 2. Ceci signifie que le système de libération de soupape n'agit pas dès que la soupape 3 atteint sa position de distribution, mais un système de frein 50 ou 60, tel que ceux décrits en référence aux figures 5 à 11, coopère avec l'élément de commande 40, de sorte que celui-ci met un certain temps prédéterminé pour libérer l'organe de retenue 30 et donc le premier élément de blocage 20a. Pour ce faire, l'élément de commande 40, qui permet le déclenchement du système de libération de soupape, en permettant le déplacement de l'organe de retenue 30 de sa position de retenue vers sa position de non-retenue, pour libérer le premier élément de blocage 20a, est réalisé avec une première partie 48 d'élément de commande et une seconde partie 49 d'élément de commande, ces deux parties étant interconnectées par un ressort 45, de préférence de faible raideur. La première partie 48 de l'élément de commande coopère avec l'élément élastique 10 et la seconde partie 49 de l'élément de commande coopère d'une part avec l'organe de retenue 30 et d'autre part avec un second élément de blocage 20b solidaire du système de frein 60. Ainsi, lorsque le dispositif est actionné, l'élément élastique 10 du système de déclenchement par l'inhalation déplace la soupape 3 de sa position de repos vers sa position de distribution, et déplace en même temps la première partie 48 de l'élément de commande, ce qui a pour effet de comprimer le ressort 45. Le ressort 45 étant beaucoup moins puissant que le ressort de l'élément élastique 10, le déplacement de la soupape 3 vers sa position de distribution n'est pas du tout ralenti ou freiné par la présence du ressort 45. Lorsque la soupape 3 atteint sa position de distribution, le ressort 45 agit alors sur la seconde partie 49 de l'élément de commande, qui déplace le second élément de blocage 20b, ce déplacement étant freiné par le système de frein 60. Ce n'est donc qu'après un certain retardement, prédéterminé par les caractéristiques du ressort 45 et du frein 60, que la seconde partie 49 de l'élément de commande 40 atteint la position dans laquelle l'organe de retenue 30 est libéré, permettant au premier élément de blocage 20a de se déplacer vers sa position de déblocage.

L'exemple de réalisation représenté sur les figures 15 à 17 utilise un système de frein pneumatique et un système de retardateur par ressort mécanique, mais on pourrait tout à fait utiliser un retardateur du type électronique ou électromécanique. Dans ce cas, le frein pourrait être remplacé par un retardateur électronique et le système de libération de soupape pourrait être remplacé par un électro-aimant qui entraîne un élément à coulisser lorsqu'il est actionné, cet élément étant l'équivalent de la deuxième partie 49 de l'élément de commande 40.

Bien que la présente invention ait été décrite en référence à plusieurs modes de réalisation de celle-ci, qui sont donnés à titres d'exemples non limitatifs, il est clair que l'homme du métier peut y apporter plusieurs modifications sans sortir du cadre de la présente invention défini par les revendications annexées.

## Revendications

1. Dispositif de distribution de produit fluide comportant un réservoir (1) contenant du produit fluide et un gaz propulseur, une valve doseuse (2), montée sur ledit réservoir, comportant une chambre de dosage et une soupape (3) mobile entre une position de distribution et une position de repos, et un système de déclenchement automatique, de préférence actionné par l'inhalation de l'utilisateur, pour actionner ladite valve, ledit système de déclenchement comportant un élément élastique (10), formé par ou solidaire d'un ressort, ledit élément élastique étant déplacé manuellement par l'utilisateur vers une position armée, dans laquelle il est maintenu sous tension, l'actionnement du système de déclenchement libérant ledit élément élastique (10) qui se déplace alors vers une position d'actionnement en exerçant une force adaptée à déplacer la soupape (3) de la valve (2) vers sa position de distribution, ledit élément élastique (10) étant ensuite ramené manuellement par l'utilisateur de sa position d'actionnement vers sa position de repos, dans laquelle il ne sollicite plus ladite soupape vers sa position de distribution, **caractérisé en ce que** le dispositif comporte un système de libération de soupape qui coopère avec le réservoir (1) ou la soupape (3) de la valve (2) de telle sorte qu'après distribution du produit contenu dans la chambre de dosage de la valve, ladite soupape (3) est libérée de la force exercée par ledit élément élastique (10) du système de déclenchement, de telle sorte qu'après l'actionnement du dispositif, ladite soupape (3) est ramenée vers sa position de repos par le ressort de rappel de la valve, indépendamment de la position dudit élément élastique.

2. Dispositif selon la revendication 1, dans lequel ledit système de libération de soupape comporte un élément de blocage (20) coopérant avec l'un parmi la soupape (3) de la valve et le réservoir (1) de produit, ledit élément de blocage (20) étant mobile entre une position de blocage, dans laquelle la soupape (3) peut être amenée dans sa position de distribution par ledit élément élastique (10) du système de déclenchement, et une position de déblocage, dans laquelle la soupape (3) est ramenée dans sa position de repos indépendamment de la position dudit élément élastique (10), ledit élément de blocage (20) étant sollicité vers sa position de déblocage après actionnement du système de déclenchement.

3. Dispositif selon la revendication 2, dans lequel le système de libération de soupape comporte un organe de retenue (30) déplaçable entre une position de retenue dans laquelle il retient ledit élément de blocage (20) dans sa position de blocage et une position de non-retenue, dans laquelle il ne retient pas ledit élément de blocage (20) dans sa position de blocage, ledit organe de retenue (30) étant déplacé vers sa position de non-retenue lorsque la soupape (3) atteint sa position de distribution.

4. Dispositif selon la revendication 3, dans lequel ledit système de libération de soupape comporte un élément de commande (40) coopérant d'une part avec la soupape (3) de la valve et d'autre part avec ledit organe de retenue (30), de telle sorte que lorsque la soupape (3) arrive dans sa position de distribution, l'élément de commande (40) permet le déplacement de l'organe de retenue (30) dans sa position de non-retenue, de sorte que l'élément de blocage (20) est déplacé vers sa position de déblocage et la soupape est ramenée dans sa position de repos par le ressort de rappel de la valve.

5. Dispositif selon la revendication 4, dans lequel ledit organe de retenue (30) est élastiquement déformable et ledit élément de commande comporte un premier diamètre interne (41) coopérant avec l'organe de retenue (30) pour empêcher sa déformation et ainsi le maintenir dans sa position de retenue, et un second diamètre interne (42) supérieur audit premier diamètre interne (41), qui coopère avec ledit organe de retenue (30) lorsque la soupape (3) atteint sa position de distribution, permettant alors la déformation dudit organe de retenue (30) vers sa position de non retenue.

6. Dispositif selon l'une quelconque des revendications 2 à 5, dans lequel ledit élément de blocage (20) comporte un dispositif de frein (50, 60) adapté à ralentir le déplacement dudit élément de blocage (20) vers sa position de déblocage, après actionnement du système de déclenchement.

7. Dispositif selon la revendication 6, dans lequel ledit dispositif de frein (50) est mécanique et comporte un élément mobile (55) relié audit élément de blocage (20) et coulissant à frottement contre ou entre un ou plusieurs organes de frein (51, 52).

8. Dispositif selon la revendication 7, dans lequel ledit élément mobile est une tige (55), de préférence dentelée, et lesdits organes de frein comportent deux roues (51, 52), de préférence en élastomère, ladite tige (55) coulissant avec frottement entre lesdites roues (51,52).

9. Dispositif selon la revendication 8, dans lequel chaque roue (51, 52) est solidaire d'un bras déformable respectif (53, 54), de telle sorte que lorsque la tige (55) coulisse entre lesdites roues, chaque bras (53, 54) se déforme de telle sorte que lesdites roues (51, 52) se rapprochent l'une de l'autre, augmentant le frottement exercée sur ladite tige (55) pour réaliser le freinage.

10. Dispositif selon la revendication 6, dans lequel ledit dispositif de frein (60) est pneumatique et/ou hydraulique.

11. Dispositif selon la revendication 10, dans lequel ledit dispositif de frein (60) comporte un piston (61) relié audit élément de blocage (20), ledit piston (61) coulissant de manière étanche dans une chambre (62), ladite chambre (62) ou ledit piston (61) étant pourvu(e) d'un petit orifice (63), de sorte que du gaz ou du liquide ne peut s'écouler que lentement dans ou hors de ladite chambre (62), assurant un déplacement lent dudit piston (61).

12. Dispositif selon l'une des revendications 3 à 5 et l'une des revendications 6 à 11, dans lequel le système de libération de soupape comporte un premier élément de blocage (20a) déplacé vers sa position de déblocage lorsque la soupape (3) atteint sa position de distribution, et un second élément de blocage (20b) pourvu d'un système de frein (50, 60), et sollicité vers sa position de déblocage par ledit premier élément de blocage (20a) lorsqu'il est en position de déblocage, de sorte que le système de frein (50, 60) n'est actionné qu'à partir du moment où la soupape (3) est dans sa position de distribution.

13. Dispositif selon la revendication 12, dans lequel ledit premier élément de blocage (20a) coopère d'une part avec le fond du réservoir (1) et d'autre part avec ledit second élément de blocage (20b), solidaire dudit frein (50, 60).

14. Dispositif selon l'une quelconque des revendications précédentes, dans lequel ledit système de libération de soupape comporte un système de retardateur adapté à libérer la soupape (3) de la valve doseuse (2) après un temps de retardement prédéterminé après que la soupape (3) a atteint sa positon de distribution.

15. Dispositif selon la revendication 14, dans lequel le système de libération de soupape comporte un premier élément de blocage (20a) retenu par un organe de retenue (30) lui-même coopérant avec un élément de commande (40), ledit élément de commande (40) comportant une première partie (48) solidaire de l'élément élastique (10) du système de déclenchement, et une seconde partie (49) coopérant d'une part avec ledit organe de retenue (30), et d'autre part avec un second élément de blocage (20b) pourvu d'un système de frein (50,60), un organe élastique (45) de faible raideur, tel qu'un ressort, étant interposé entre lesdites première et seconde parties (48, 49) de l'élément de commande (40), de sorte que ledit premier élément de blocage (20a) ne peut se déplacer vers sa position de déblocage qu'après un temps de retardement prédéterminé pour permettre à ladite soupape (3) de la valve (2) de retourner vers sa position de repos, ledit temps de retardement correspondant au temps nécessaire audit second élément de blocage (20b) pour se déplacer vers sa position de déblocage, contre ledit système de frein (50, 60), sous l'effet dudit organe élastique (45), pour déplacer ainsi ladite seconde partie (49) de l'élément de commande (40) dans la position dans laquelle l'organe de retenue (30) peut se déformer dans sa position de non-retenue, pour permettre au premier élément de blocage (20a) de se déplacer vers sa position de déblocage.

16. Dispositif selon la revendication 14, dans lequel ledit système retardateur est électronique ou électro-mécanique.

## Claims

1. A fluid dispenser device comprising a reservoir (1) containing fluid and a propellant gas, a metering valve (2) mounted on said reservoir and comprising a metering chamber and a valve member (3) mounted to move between a dispensing position and a rest position, and an automatic trigger system for actuating said valve and that is preferably actuated by the user inhaling, said trigger system comprising a resilient element (10) formed by or secured to a spring, said resilient element being moved manually by the user into a cocked position in which it is held under tension, actuation of the trigger system releasing said resilient element (10) which then moves to an actuating position while exerting a force adapted to moving the valve member (3) of the valve (2) towards its dispensing position, said resilient element (10) then being returned manually by the user from its actuating position to its rest position member in which it no longer urges said valve member towards its dispensing position, said fluid dispenser device being **characterized in that** it further comprises a valve member release system which co-operates with the reservoir (1) or with the valve member (3) of the valve (2) so that, after dispensing the fluid contained in the metering chamber of the valve, said valve member (3) is released from the force exerted by said resilient element (10) of the trigger system, so that, after the device is actuated, said valve member (3) is returned to its rest position by the return spring of the valve, independently of the position of said resilient element.

2. A device according to claim 1, in which said valve member release system comprises a locking element (20) co-operating with one of the valve member (3) and the fluid reservoir (1), said locking element (20) being mounted to move between a locking position, in which the valve member (3) can be brought into its dispensing position by said resilient element (10) of the trigger system, and an unlocking position, in which the valve member (3) is brought into its rest position independently of the position of said resilient element (10), said locking element (20) being urged to its unlocking position after the trigger system has been actuated.

3. A device according to claim 2, in which the valve member release system includes a retaining member (30) that can be moved from a retaining position in which it retains said locking element (20) in its locking position, and a non-retaining position in which it does not retain said locking element (20) in its locking position, said retaining member (30) being moved towards its non-retaining position when the valve member (3) reaches its dispensing position.

4. A device according to claim 3, in which said valve member release system includes a control element (40) co-operating firstly with the valve member (3) and secondly with the retaining member (30) so that when the valve member (3) reaches its dispensing position, the control element (40) makes it possible to move the retaining member (30) to its non-retaining position so that the locking element (20) is moved into its unlocking position, and the valve member is returned to its rest position by the return spring of the valve.

5. A device according to claim 4, in which said retaining member (30) is resiliently deformable and said control element includes a first inside diameter (41) co-operating with the retaining member (30) to prevent it from deforming and thus to hold it in its retaining position, and a second inside diameter (42) greater than said first inside diameter (41), which co-operates with said retaining member (30) when the valve member (3) reaches its dispensing position, then making it possible to deform said retaining member (30) towards its non-retaining position.

6. A device according to any one of claims 2 to 5, in which said locking element (20) includes a brake device (50, 60) adapted to slow down the movement of said locking element (20) towards its unlocking position, after the trigger system has been actuated.

7. A device according to claim 6, in which said brake device (50) is mechanical and includes a moving element (55) connected to said locking element (20), and that slides with friction against or between one or more brake members (51, 52).

8. A device according to claim 7, in which said moving element is a preferably serrated rod (55), and said brake members comprise two preferably elastomer wheels (51, 52), said rod (55) sliding with friction between said wheels (51, 52).

9. A device according to claim 8, in which each wheel (51 52) is secured to a respective deformable arm (53, 54) so that when the rod (55) slides between said wheels, each arm (53, 54) deforms so that said wheels (51, 52) come towards each other, thereby increasing the friction exerted on said rod (55) to perform the braking.

10. A device according to claim 6, in which said brake device (60) is pneumatic and/or hydraulic.

11. A device according to claim 10, in which said brake device (60) includes a piston (61) connected to said locking element (20), said piston (61) sliding in leaktight manner in a chamber (62), said chamber (62) or said piston (61) being provided with a small orifice (63) so that gas or liquid can flow only slowly into or out from said chamber (62), thereby causing said piston (61) to move slowly.

12. A device according to any one of claims 3 to 5, and any one of claims 6 to 11, in which the valve member release system comprises a first locking element (20a) moved to its unlocking position when the valve member (3) reaches its dispensing position, and a second locking element (20b) provided with a brake system (50, 60), and urged into its unlocking position by said first locking element (20a) when it is in the unlocking position, so that the brake system (50, 60) is actuated only once the valve member (3) is in its dispensing position.

13. A device according to claim 12, in which said first locking element (20a) co-operates firstly with the endwall of the reservoir (1) and secondly with said second locking element (20b) which is secured to said brake (50, 60).

14. A device according to any preceding claim, in which said valve member release system includes a delay system adapted to release the valve member (3) of the metering valve (2) after a predetermined delay time after the valve member (3) has reached its dispensing position.

15. A device according to claim 14, in which the valve member release system comprises a first locking element (20a) retained by a retaining member (30) itself co-operating with a control element (40), said control element (40) having a first portion (48) secured to the resilient element (10) of the trigger system, and a second portion (49) co-operating firstly with said retaining member (30), and secondly with a second locking element (20b) provided with a brake system (50, 60), a resilient member (45) of low stiffness, such as a spring, being interposed between said first and second portions (48, 49) of the control element (40), so that said first locking element (20a) can move towards its unlocking position only after a delay time predetermined to enable said valve member (3) of the valve (2) to return to its rest position, said delay time corresponding to the time necessary for the second locking element (20b) to move into its unlocking position, against said brake system (50, 60) under drive from said resilient member (45), thereby moving said second portion (49) of the control element (40) into the position in which the retaining member (30) can deform into its non-retaining position, so as to enable the first locking element (20a) to move into its unlocking position.

16. A device according to claim 14, in which said delay system is electronic or electromechanical.

## Patentansprüche

1. Vorrichtung zum Ausgeben eines Fluiderzeugnisses, aufweisend einen Vorratsbehälter (1), der ein Fluiderzeugnis und ein Antriebsgas enthält, ein Dosierventil (2), welches auf dem Vorratsbehälter angebracht ist und eine Dosierkammer sowie ein bewegliches Ventilorgan (3) umfasst, das zwischen einer Ausgabestellung und einer Ruhestellung beweglich ist, und ein automatisches Auslösungssystem, das bevorzugt durch Inhalation durch den Benutzer betätigt wird, um das Ventil zu betätigen, wobei das Auslösungssystem ein elastisches Element (10) umfasst, das durch eine Feder gebildet oder fest mit einer Feder verbunden ist, wobei das elastische Element manuell durch den Nutzer in Richtung auf eine Entsicherungsstellung verschoben ist, in welcher es unter Spannung steht, wobei die Betätigung des Auslösungssystems das elastische Element (10) freigibt, welches sich dadurch in eine Betätigungsstellung verschiebt, in welcher es eine Kraft ausübt, die hinreicht, das Ventilorgan (3) des Ventils (2) in seine Austragstellung zu verschieben, wobei das elastische Element (10) daraufhin manuell durch den Nutzer aus seiner Betätigungsstellung in seine Ruhestellung überführt wird, in welcher es das Ventilorgan nicht weiterhin in Richtung auf die Ausgabestellung vorspannt, **dadurch gekennzeichnet, dass** die Vorrichtung ein Freigabesystem für das Ventilorgan umfasst, das mit dem Vorratsbehälter (1) bzw. dem Ventilorgan (3) des Ventils (2) derart zusammenwirkt, dass nach Austragen des in der Dosierkammer des Ventils enthaltenen Erzeugnisses das Ventilorgan (3) von der Kraft freigegeben wird, die durch das elastische Element (10) des Auslösungssystems ausgeübt wird, so dass nach Betätigung der Vorrichtung das Ventilorgan (3) in Richtung auf seine Ruhestellung durch die Rückstellfeder des Ventils überführt wird, und zwar unabhängig von der Stellung des elastischen Elements.

2. Vorrichtung nach Anspruch 1, wobei das Freigabesystem für das Ventilorgan ein Blockierelement (20) umfasst, welches entweder mit dem Ventilorgan (3) des Ventils oder dem Vorratsbehälter (1) für das Erzeugnis zusammenwirkt, wobei das Blockierelement (20) zwischen einer Blockadestellung, in welcher das Ventilorgan (3) in seine Ausgabestellung durch das elastische Element (10) des Auslösungssystems gebracht werden kann, und einer Blockadeaufhebestellung beweglich ist, in welcher das Ventilorgan (3) in seiner Ruhestellung unabhängig von der Stellung des elastischen Elements (10) überführbar ist, wobei das Blockierelement (20) in Richtung auf seine Blockadeaufhebestellung nach Betätigung des Auslösungssystems vorgespannt ist.

3. Vorrichtung nach Anspruch 2, wobei das System zur Freigabe des Ventilorgans ein Rückhalteorgan (30) umfasst, welches zwischen einer Rückhaltestellung, in welcher es das Blockierelement (20) in seiner Blockierstellung rückhält, und einer Nichtrückhaltestellung verschiebbar ist, in welcher es das Blockierelement (20) nicht in seiner Blockadestellung rückhält, wobei das Rückhalteorgan (30) in Richtung auf seine Nichtrückhaltestellung verschoben wird, wenn das Ventilorgan (3) seine Austragstellung erreicht.

4. Vorrichtung nach Anspruch 3, wobei das System zur Freigabe des Ventilorgans ein Steuerelement (40) umfasst, welches einerseits mit dem Ventilorgan (3) des Ventils und andererseits mit dem Rückhalteorgan (30) so zusammenwirkt, dass dann, wenn das Ventilorgan (3) seine Austragstellung erreicht, das Steuerelement (40) die Verschiebung des Rückhalteorgans (30) in seine Nichtrückhaltestellung derart erlaubt, dass das Blockierelement (20) in Richtung auf seine Blockadeaufhebestellung verschoben wird und das Ventilorgan in seine Ruhestellung durch die Rückstellfeder des Ventils überführt wird.

5. Vorrichtung nach Anspruch 4, wobei das Rückhalteorgan (30) elastisch verformbar ist, und wobei das Steuerelement einen ersten Innendurchmesser (41), der mit dem Rückhalteelement (30) zusammenwirkt, um seine Verformung zu unterbinden und dadurch seine Rückhaltestellung beizubehalten, und einen zweiten Innendurchmesser (42) aufweist, der größer ist als der erste Innendurchmesser (41) und mit dem Rückhalteorgan (30) zusammenwirkt, wenn das Ventilorgan (3) seine Austragstellung erreicht, wodurch die Verformung des Rückhalteorgans (30) in Richtung auf seine Nichtrückhaltestellung möglich ist.

6. Vorrichtung nach einem der Ansprüche 2 bis 5, wobei das Blockierelement (20) eine Bremseinrichtung (50, 60) umfasst, die dazu ausgelegt ist, die Verschiebung des Blockierelements (20) in Richtung auf die Blockadeaufhebestellung nach einer Betätigung des Auslösungssystems zu verlangsamen.

7. Vorrichtung nach Anspruch 6, wobei die Bremseinrichtung (20) eine mechanische Bremseinrichtung ist und ein bewegliches Element (55) umfasst, das mit dem Blockierelement (20) verbunden ist und unter Reibung in Bezug auf ein oder mehrere Bremsorgane (51, 52) sowie zwischen diesen gleitet.

8. Vorrichtung nach Anspruch 7, wobei das bewegliche Element eine Stange (55), bevorzugt eine Zahnstange ist, und wobei die Bremsorgane zwei Räder (51, 52), bevorzugt aus einem Elastomer umfassen, wobei die Stange (55) unter Reibung zwischen den Rädern (51, 52) gleitet.

9. Vorrichtung nach Anspruch 8, wobei jedes Rad (51, 52) fest mit einem jeweiligen verformbaren Arm (53, 54) derart verbunden ist, dass die Stange (55) zwischen den Rädern gleitet, wobei jeder Arm (53, 54) sich derart verformt, dass die Räder (51, 52) sich gegenseitig unter Vergrößerung der Reibung nähern, die auf die Stange (55) ausgeübt ist, um den Bremsvorgang zu verwirklichen.

10. Vorrichtung nach Anspruch 6, wobei die Bremseinrichtung (60) eine pneumatische und/oder hydraulische Bremse ist.

11. Vorrichtung nach Anspruch 10, wobei die Bremseinrichtung (60) einen Kolben (61) umfasst, der mit dem Blockierelement (20) verbunden ist, wobei der Kolben (61) in einer Kammer 62 dicht gleitet, wobei die Kammer (62) bzw. der Kolben (61) mit einer kleinen Öffnung (63) so versehen ist, dass Gas oder Flüssigkeit ausschließlich langsam in der Kammer (62) oder aus dieser heraus unter Gewährleistung einer langsamen Verschiebung des Kolbens (61) zu strömen vermag.

12. Vorrichtung nach einem der Ansprüche 3 bis 5 und einem der Ansprüche 6 bis 11, wobei das System zur Freigabe des Ventilorgans ein erstes Blockierelement (20a) umfasst, das in Richtung auf seine Blockierstellung verschoben wird, wenn das Ventilorgan (3) seine Ausgabestellung erreicht, und ein zweites Blockierelement (20b), das mit einem Bremssystem (50, 60) versehen ist und in Richtung auf seine Blockadeaufhebungsstellung durch das erste Blockierelement (20a) vorgespannt ist, wenn es sich in der Blockadeaufhebungsstellung befindet, so dass das Bremssystem (50, 60) ausschließlich ab dem Zeitpunkt betätigt wird, in welchem das Ventilorgan (30) in seiner Ausgabestellung befindet.

13. Vorrichtung nach Anspruch 12, wobei das erste Blockierelement (20a) einerseits mit dem Boden des Vorratsbehälters (1) und andererseits mit dem zweiten Blockierelement (20b) zusammenwirkt, das mit der Bremse (50, 60) fest verbunden ist.

14. Vorrichtung nach einem der vorangehenden Ansprüche, wobei das System zur Freigabe des Ventilelements ein Verzögerungssystem umfasst, das dazu ausgelegt ist, das Ventilelement (3) und das Dosierventil (2) nach einer vorbestimmten Verzögerungszeit freizugeben, nachdem das Ventilorgan (3) seine Ausgabestellung erreicht hat.

15. Vorrichtung nach Anspruch 14, wobei das System zur Freigabe des Ventilorgans ein erstes Blockierelement (20a) umfasst, das durch ein Rückhalteelement (30) rückgehalten ist, welches seinerseits mit einem Steuerelement (40) zusammenwirkt, wobei das Steuerelement (40) einen ersten Teil (48) umfasst, der mit dem elastischen Element (10) des Auslösungssystems fest verbunden ist, und einen zweiten Teil (49), der einerseits mit dem Rückhalteorgan (30) und andererseits mit einem zweiten Blockierelement (20b) zusammenwirkt, das mit einem Bremssystem (59, 60) versehen ist, ein elastisches Organ (45) geringer Dicke, wie etwa eine Feder, das zwischen den ersten und zweiten Teilen (48, 49) des Steuerelements (40) derart angeordnet ist, dass es sich ausschließlich nach einer vorbestimmten Verzögerungszeit in seine Blockadeaufhebungsstellung verschieben kann, um zu ermöglichen, dass das Ventilorgan (3) des Ventils (2) in seiner Ruhestellung rückkehrt, wobei die Verzögerungszeit einer Zeit entspricht, die erforderlich ist, dass das zweite Blockierelement (20b) sich in Richtung auf seine Blockadeaufhebungsstellung verschieben kann, und zwar entgegen dem Bremssystem (50, 60) unter Einwirkung des elastischen Elements (45), um dadurch den zweiten Teil (49) des Steuerteils (40) in die Stellung zu verschieben, in welcher das Rückhalteorgan (30) sich in seine Nichtrückhaltestellung verformen kann, um es dem Blockierelement (20a) zu ermöglichen, in seine Blockadeaufhebungsstellung zu verschieben.

16. Vorrichtung nach Anspruch 14, wobei das Verzögerungssystem ein elektronisches oder ein elektromechanisches Verzögerungssystem ist.
